# EUROPEAN PATENT SPECIFICATION

(11) **EP 1 450 850 B1**
(45) Date of publication and mention of the grant of the patent: **22.09.2010**
(21) Application number: 02802520.3
(22) Date of filing: 01.11.2002
(51) Int. Cl.: A61K 38/22, A61K 38/04, C07K 14/575, A61P 35/00, A61P 31/18, A61P 31/14, A61P 31/20

(54) **METHOD OF ADMINISTERING A THYMOSIN ALPHA 1 PEPTIDE**
VERFAHREN ZUR VERABREICHUNG EINES THYMOSIN-ALPHA 1 PEPTIDS
METHODE D'ADMINISTRATION D'UN PEPTIDE ALPHA 1 DE LA THYMOSINE

(30) Priority: 01.11.2001 US 330874 P
(43) Date of publication of application: 01.09.2004
(73) Proprietor: Sciclone Pharmaceuticals, Inc., San Mateo, CA 94404 (US)
(72) Inventor: RUDOLPH, Alfred, R., Los Altos Hills, CA 94022 (US); TUTHILL, Cynthia, W., Menlo Park, CA 94025 (US)
(74) Representative: von Füner, Nicolai
(86) International application number: PCT/US2002/035093
(87) International publication number: WO 2003/037366

(56) References cited:
- US-A- 5 468 729
- US-A- 5 849 696
- US-A- 5 888 980

## Description

The present invention relates to the use of a Thymosin alpha 1 peptide for the preparation of a pharmaceutical form for treatment of cancer, HIV or hepatitis.

Thymosin alpha 1 (sometimes referred to as TA1) is a 28-amino acid thymic peptide with immunomodulatory properties, homologous to a natural product originally isolated from thymosin fraction 5 of calf thymus. Its biological effects include augmentation of T lymphocyte function and include modulation of interleukin-2 (IL-2), stimulation of interferon-γ production, induction of T lymphocytes and NK cell activity, and stimulation of thymopoiesis. Thymosin alpha 1 also has been shown to up-regulate MHC Class I expression.

Thymosin alpha 1 has previously been suggested for use in certain treatments of cancer, Hepatitis B and C, HIV, e.g., by subcutaneous injection twice weekly. There remains a need in the art for improved methods of administering Thymosin alpha 1.

US-A-5468729 discloses a method of treating autoimmune hepatitis which comprises administering to a patient in need of such treatment an immunomodulatory amount of Thymosin α₁ and an anti-inflammatory amount of a corticosteroid. This document discloses the use of Th α₁ twice weekly at a dosage within the range of about 0.4 - 4 mg (e.g., about 1.6 mg). Injections were continued for approximately 5 weeks.

US-A-5849696 discloses a method for the treatment of a mammal infected with hepatitis C virus comprising administering to said mammal an immune system potentiating dose of at least one thymosin selected from the group consisting of Thymosin Fraction Five, Th α₁, and fragments of Th α₁. Said document discloses that the dosages and length of Th α₁ treatment can be flexible, but for a typical human patient an administration regimen of twice weekly subcutaneous injection of about 1.5 to about 1.7 mg of Th α₁ is convenient. Additionally, this document discloses that a pharmaceutical dosage unit of thymosin can be from about 900 to about 1200 mg/m² body surface area in a pharmaceutically acceptable carrier.

US-A-5888980 discloses a composition comprising thymosin and naloxone and a composition comprising Th α₁ and melanocyte-stimulating hormone inhibiting factor (MIF) as well as a method for enhancing immune competence comprising administering to said patient a therapeutically-effective amount of said compositions. Said document discloses that the therapeutic human dosage of the compositions is from about 0.02 to about 20 mg/m², based on the body surface area, or alternatively from about 0.01 mg to about 100 mg/Kg of body weight per day or higher.

In accordance with the present invention, a Thymosin alpha 1 peptide is used for the preparation of a pharmaceutical form for administration to a patient in need of immune stimulation for treatment of cancer, HIV or hepatitis in said patient so that the Thymosin alpha 1 is continuously administered to the patient at a rate within a range of 0.0001-0.1 mg/hr/kg patient body weight, so as to continuously maintain an immune stimulating effective amount of said Thymosin alpha 1 in the patient during a treatment period of at least 6 hours.

The present invention is based on a discovery that maintaining immune stimulating-effective amounts of a TA1 peptide in a patient's circulatory system during a treatment period provides a substantial improvement in the immune stimulating effect of the TA1 peptide.

The invention is applicable to TA1 peptides including naturally occurring TA1 as well as synthetic TA1 and recombinant TA1 having the amino acid sequence of naturally occurring TA1, amino acid sequences substantially similar thereto, or an abbreviated sequence form thereof, and their biologically active analogs having substituted, deleted, elongated, replaced, or otherwise modified sequences which possess bioactivity substantially similar to that of TA1, e.g., a TA1 derived peptide having sufficient amino acid homology with TA1 such that it functions in substantially the same way with substantially the same activity as TA1.

Because the plasma half-life of subcutaneously injected TA1 is only about two hours, according to one embodiment, a TA1 peptide such as TA1 is administered to a patient in need of immune stimulation so as to continuously maintain an immune stimulating-effective amount of the TA1 peptide in the patient's circulatory system during a substantially longer treatment period. Although much longer treatment periods are contemplated in accordance with the present invention, embodiments of the invention include continuously maintaining an immune stimulating-effective amount of the TA1 peptide in the patient's circulatory system during treatment periods of at least 6, 10, 12 hours, or longer. In other embodiments, treatment periods are for at least a day, and even for a plurality of days, e.g., a week or longer. However, it is contemplated that treatments, as defined above, in which immune stimulating-effective amounts of the TA1 peptide are continuously maintained in the patient's circulatory system, may be separated by non-treatment periods of similar or different durations.

In accordance with one embodiment, the TA1 peptide is continuously infused into a patient, e.g., by intravenous infusion, during the treatment period, so as to continuously maintain an immune stimulating-effective amount of the TA1 peptide in the patient's circulatory system. The infusion may be carried out by any suitable means, such as by minipump.

Alternatively, an injection regimen of the TA1 peptide can be maintained so as to continuously maintain an immune stimulating-effective amount of the TA1 peptide in the patient's circulatory system. Suitable injection regimens may include an injection every 1, 2, 4, 6 hours, so as to continuously maintain the immune stimulating- effective amount of the Thymosin alpha 1 peptide in the patient's circulatory system during the treatment period.

Although it is contemplated that during continuous infusion of the TA1 peptide, administration will be for a substantially longer duration, according to one embodiment the continuous infusion of the TA1 peptide is for a treatment period of at least 1 hour. More preferably, continuous infusion is carried out for longer periods, such as for periods of at least 6, 8, 10, 12 hours, or longer. In other embodiments, continuous infusion is for at least one day, and even for a plurality of days such as for one week or more.

Immune stimulating-effective amounts of a TA1 peptide may be continuously maintained in a patient's circulatory system by administering the TA1 peptide to the patient at a rate within a range of 0.0001 - 0.1 mg/hr/Kg patient body weight. Preferred administration rates are within a range of 0.0003 - 0.03 mg/hr/Kg patient body weight.

In preferred embodiments, the TA1 peptide is present in a pharmaceutically acceptable liquid carrier, such as water for injection, saline in physiological concentrations, or similar.

The invention may be utilized for treatment of any patient in need of immune stimulation, including cancer patients, HIV patients, and patients having various forms of hepatitis, including Hepatitis B and Hepatitis C. For example, the invention may be utilized to promote bone marrow recovery in cancer patients following chemotherapy. The invention may be particularly useful for addition of TA1 to chemoimmunotherapy for increased survival in melanoma and hepatocellular carcinoma (HCC)
patients, and for reduction of haematological toxicity in lung cancer.

In the following examples a continuous infusion of TA1 was evaluated in a cancer therapy model with the use of surgically implanted osmotic minipumps, which deliver fluids at a constant flow rate for 5 days. Rats were given 5-fluorouracil (5-FU) to cause immune suppression, and then treated with injected or infused TA1 8 days later (the nadir of white cell count after 5-FU). Treatment groups, 8 rats each, were: control (minipumps with saline); low dose TA1 (0.2 mg/Kg sc injection; empty minipumps); high dose TA1 (3.5 mg/Kg sc injection; empty minipumps); and high dose infused TA1 (3.5 mg/Kg infused by minipumps). Immune parameters were determined at baseline and 8 days after 5-FU treatment (day 1 of TA1 treatment), and also at 5, 12, 20, and 27 days after TA1 treatment.

### Example 1

10 week old rats, weighing 250-300 g, received 100 mg/kg 5-fluorouracil (5-FU) for immune suppression.

8 days after 5-FU treatment, rats were randomly assigned to one of the following groups (n=8):
· Control (saline in minipump)
· Low dose TA1, injected s.c. at 0.2 mg/Kg (with empty minipumps)
· High dose TA1, injected s.c. at 3.5 mg/Kg (with empty minipumps)
· Continuous infusion TA1, provided by minipump at 3.5 mg/Kg/5 days

Immune parameters were determined at baseline and 8 days after 5-FU treatment (day 1 of TA1 treatment), and also at 5, 12, 20, and 27 days after TA1 treatment.

The evaluations included NK activity (LDH released from YAC-1 cells after 4h exposure to PBMC), total leukocyte number (judged by physical cytofluorimetric parameters, after verifying the specificity by monoclonal antibody), total lymphocyte number (CD3+ by flow cytometry), and activated lymphocytes (CD25+CD3+ by flow cytometry).

NK activity was 42 ± 5 % at baseline and was depressed to 9 ± 2 % after 5-FU. Low dose TA1 treatment lead to a significant recovery of NK activity after 12 days, while high dose TA1 achieved significant recovery in only 5 days. Continuous infusion of TA1, however, was able to double the response at 5 days, to 32 ± 4% (versus 16 ± 2 for high dose injected, 12 ± 3 low dose injected, and 11 ± 1 control). Only animals treated with TA1 by continuous infusion had a complete recovery of NK activity to baseline levels.

Total white blood cell count, as determined by morphology, was depressed from 14,590 ± 2,071 cells/mm³ to 2,597 ± 582 after treatment with 5-FU. Low or high dose TA1 treatment by injection trended towards a sooner increase in recovery compared to untreated animals. Continuous infusion of TA1, however, provided statistically significant and complete recovery to baseline levels after only 5 days.

Activated lymphocytes (CD3+CD25+) were not decreased significantly by 5-FU treatment (from 65 ± 21 cells/mm³ to 37 ± 10), however, the levels were dramatically increased 12 and 20 days after high dose TA1 treatment (297 ± 136 and 321 ± 75 cells/mm³ vs 166 ± 70 and 212 ± 77 cells/mm³, respectively). TA1 provided by continuous infusion lead to an even greater increase, to 422 ± 105 and 446 ± 73 cells/mm³.

### Example 2

10 week old rats, weighing 250 - 300 g, received 100 µ/kg 5-FU for immune suppression.

8 days after 5-FU treatment, rats were randomly assigned to one of the following groups (n=15):
- Control (saline in minipump)
- High dose TA1, injected s.c. at 3.5 mg/Kg (with empty minipumps)
- Continuous infusion TA1, provided by minipump at 3.5 mg/Kg/5 days

Immune parameters were determined at baseline and 8 days after 5-FU treatment (day 1 of TA1 treatment), and also at 5 and 14 days after TA1 treatment.

The evaluations included total leukocyte number (judged by physical cytofluorimetric parameters, after verifying the specificity by monoclonal antibody), granulocytes (flow cytometry using FITC anti rat granulocyte HIS-48), total lymphocyte number (CD3+ by flow cytometry), T helper lymphocytes (CD4+ by flow cytometry), activated lymphocytes (CD25+CD3+ by flow cytometry), and cytokine expression in plasma (IL-2 and IFN-γ by ELISA).

After determining in Example 1 that TA1 provided by continuous infusion compared to s.c. injection had a dramatic effect on the total number of leukocytes, it was of interest to determine which type of white blood cell was responsible for the increase. Granulocytes appear to be the subset of white blood cells that are most affected by TA1 provided by continuous infusion. The number of granulocytes was decreased after 5-FU from 4,485 ± 1,116 to 1,249 ± 432. Treatment with TA1 resulted in an increase to 14,652 ± 2,463 within 5,days (compared to 9,924 ± 3,218 with TA1 by injection or 6,954 ± 1,519 with no TA1), and this level was still the highest after 14 days.

Interestingly, there was one animal in this study which was provided TA1 by BOTH injection (of 3.5 mg/Kg) and by continuous infusion (of another 3.5 mg/Kg). Not only was this animal healthy and vigorous, with no obvious adverse events, but the TA1 effects on the immune parameters measured were even greater than those in the other animals. For granulocytes, this study animal had a greatly increased level of 19,376 cells/mm³ after 5 days, compared to the mean of 14,652 ± 2,463 in the other infused animals.

The number of total lymphocytes (CD3+) was dramatically decreased by 5-FU treatment (from 10,904 ± 1,973 cells/mm³ to 1,740 ± 560). Treatment with TA1 allowed for a recovery to baseline levels, which occurred after only 5 days when TA1 was provided by continuous infusion but was not seen until 14 days for injected TA1.

The animal that had TA1 provided by both injection and infusion had levels of lymphocytes which were not much different from the other animals (9,765 cells/mm³ compared to the mean of 9,644 ± 961), but the percentage of these lymphocytes which were activated was greatly increased (from 428 ± 89, or 4% of lymphocytes, for the animals with TA1 by infusion, to 976, or 10% of lymphocytes, for the animal which had TA1 in a high dose injection followed by infusion).

T helper lymphocytes (CD3+CD4+) were also depressed by treatment with 5-FU, from 5,411 ± 1,084 cells/mm³ to 1,710 ± 449. These depressed levels of T cells did not increase without treatment with TA1 for the 14 days of the experiment. By contrast with the results seen for granulocytes, in which TA1 provided by continuous infusion was superior to TA1 provided by injection for recovery of cell numbers, TA1 provided by either delivery method was sufficient to return the levels of T helper cells to baseline.

Since TA1 provided either by injection or by continuous infusion lead to an increase in CD4+ T helper lymphocytes, it was of interest to determine whether this increase was due to an effect on the Th1 or the Th2 subset of T helper cells. Previous *in vitro* and *in vivo* data have demonstrated that TA1 increases the Th1 subset of T cells, and in this study the same effect was seen. Providing TA1 by continuous infusion lead to an even greater increase in the plasma level of the Th1 cytokine IL-2 than was seen after s.c. injection (42 ± 7 pg/ml 14 days after TA1 by continuous infusion, compared to 21 ± 16 for injected TA1 and 10 ± 16 for control animals).

Treatment by TA1 lead to an increase in the Th1 cytokine IL-2, and TA1 allows for an increase in another Th1 cytokine, IFN-γ. Although the levels are low, by 5 days after treatment, s.c. injected TA1 lead to higher plasma levels of IFN-γ. By 14 days after treatment the animals with TA1 provided by continuous infusion had the highest levels (14 ± 5 pg/ml compared to 10 ± 1 by injection or 8 ± 8 for control).

The animal which received TA1 by both injection and continuous infusion had even greater levels of both of the Th1 cytokines measured, especially IFN-γ, which was 45 pg/ml after 14 days, compared to 14 ± 5 pg/ml for the other animals.

### CONCLUSIONS:

- Maintenance of a constant level of TA1 over a plurality of days in the circulation increases the measured immunological effects.
- This dosage regimen leads to unexpected positive effects on granulocytes, as well as the positive effects on monocytes seen after injection of TA1.
- No adverse events were observed, even at doses of TA1 15 times higher than usual (and in one animal, at doses 30 times higher than usual).

## Claims

1. Use of a Thymosin alpha 1 peptide for the preparation of a pharmaceutical form for administration to a patient in need of immune stimulation for treatment of cancer, HTV or hepatitis in said patient so that the Thymosin alpha 1 is continuously administered to the patient at a rate within a range of 0.0001-0.1 mg/hr/kg patient body weight, so as to continuously maintain an immune stimulating-effective amount of said Thymosin alpha 1 in the patient during a treatment period of at least 6 hours.

2. The use according to claim 1 wherein said treatment period is at least 8 hours.

3. The use according to claim 1 wherein said treatment period is at least 10 hours.

4. The use according to claim 1 wherein said treatment period is at least 12 hours.

5. The use according to claim 1 wherein said treatment period is at least one day.

6. The use according to claim 1 wherein said treatment period is one week or more.

7. The use according to claim 1 wherein said Thymosin alpha 1 is present in a pharmaceutically acceptable liquid carrier.

8. The use according to claim 1 wherein said Thymosin alpha 1 is administered to said patient at a rate of 0.0003-0.03 mg/hr/Kg patient body weight.

9. The use according to claim 1 wherein said continuous administration comprises continuous infusion of said Thymosin alpha 1.

10. The use according to claim 9 wherein said continuous infusion comprises continuous intravenous infusion.

11. The use according to claim 9 wherein said infusion is carried out by minipump.

12. The use according to claim 10 wherein said intravenous infusion is carried out by minipump.

13. The use according to claim 11 wherein said minipump is an osmotic minipump.

14. The use according to claim 11 wherein said minipump is implanted in said patient.

15. The use according to claim 1 wherein said patient is a cancer patient.

16. The use according to claim 1 for treatment of cancer in said patient.

17. The use according to claim 1 for treatment of HIV in said patient.

18. The use according to claim 1 for treatment of hepatitis B in said patient.

19. The use according to claim 1 for treatment of hepatitis C in said patient.

## Patentansprüche

1. Verwendung eines Thymosin α 1-Peptids für die Herstellung einer Arzneimittelform zur Verabreichung an einen Patienten, der einer Immunstimulierung für die Behandlung von Krebs, HIV oder Hepatitis bedarf, an diesen Patienten auf eine Weise, daß das Thymosin α 1 diesem in einer Dosis in einem Bereich von 0,0001 - 0,1 mg/hr/kg des Körpergewichts des Patienten kontinuierlich verabreicht wird, um im Patienten während einer Behandlungsdauer von wenigstens 6 Stunden eine für die Immunstimulierung wirksame Menge an Thymosin α 1 kontinuierlich aufrechtzuerhalten.

2. Verwendung nach Anspruch 1, wobei die Behandlungsdauer wenigstens 8 Stunden beträgt.

3. Verwendung nach Anspruch 1, wobei die Behandlungsdauer wenigstens 10 Stunden beträgt.

4. Verwendung nach Anspruch 1, wobei die Behandlungsdauer wenigstens 12 Stunden beträgt.

5. Verwendung nach Anspruch 1, wobei die Behandlungsdauer wenigstens einen Tag beträgt.

6. Verwendung nach Anspruch 1, wobei die Behandlungsdauer eine Woche oder mehr beträgt.

7. Verwendung nach Anspruch 1, wobei das Thymosin α 1 in einem pharmazeutisch verträglichen flüssigen Träger vorliegt.

8. Verwendung nach Anspruch 1, wobei das Thymosin α 1 dem Patienten in einer Dosis vom 0,0003 - 0,03 mg/hr/kg Körpergewicht des Patenten verabreicht wird.

9. Verwendung nach Anspruch 1, wobei die kontinuierliche Verabreichung die kontinuierliche Infusion des Thymosin α 1 umfaßt.

10. Verwendung nach Anspruch 9, wobei die kontinuierliche Infusion die kontinuierliche intravenöse Infusion umfaßt.

11. Verwendung nach Anspruch 9, wobei die Infusion mit Hilfe einer Minipumpe durchgeführt wird.

12. Verwendung nach Anspruch 10, wobei die intravenöse Infusion mit Hilfe einer Minipumpe durchgeführt wird.

13. Verwendung nach Anspruch 11, wobei die Minipumpe eine Osmoseminipumpe ist.

14. Verwendung nach Anspruch 11, wobei die Minipumpe im Patienten implantiert ist.

15. Verwendung nach Anspruch 1, wobei der Patient ein Krebspatient ist.

16. Verwendung nach Anspruch 1 zur Behandlung von Krebs bei einem Patienten.

17. Verwendung nach Anspruch 1 zur Behandlung von HIV bei einem Patienten.

18. Verwendung nach Anspruch 1 zur Behandlung von Hepatitis B bei einem Patienten.

19. Verwendung nach Anspruch 1 zur Behandlung von Hepatitis C bei einem Patienten.

## Revendications

1. Utilisation d'un peptide de thymosine alpha 1 pour la préparation d'une forme pharmaceutique destinée à l'administration à un patient manifestant un besoin de stimulation immunitaire pour le traitement du cancer, du VIH ou de l'hépatite dans ledit patient, de telle sorte que l'on administre la thymosine alpha 1 en continu au patient à un débit dans la plage de 0,0001 - 0,1 mg/h/kg de poids du corps du patient, de façon à maintenir en continu une quantité efficace pour la stimulation immunitaire de ladite thymosine alpha 1 dans le patient au cours d'une période de traitement d'au moins 6 heures.

2. Utilisation selon la revendication 1, dans laquelle ladite période de traitement s'élève à au moins 8 heures.

3. Utilisation selon la revendication 1, dans laquelle ladite période de traitement s'élève à au moins 10 heures.

4. Utilisation selon la revendication 1, dans laquelle ladite période de traitement s'élève à au moins 12 heures.

5. Utilisation selon la revendication 1, dans laquelle ladite période de traitement s'élève à au moins une journée.

6. Utilisation selon la revendication 1, dans laquelle ladite période de traitement s'élève à une semaine ou plus.

7. Utilisation selon la revendication 1, dans laquelle ladite thymosine alpha 1 est présente dans un support liquide pharmaceutiquement acceptable.

8. Utilisation selon la revendication 1, dans laquelle ladite thymosine alpha 1 est administrée audit patient à un débit de 0,0003 - 0,03 mg/h/kg de poids du corps du patient.

9. Utilisation selon la revendication 1, dans laquelle ladite administration en continu comprend la perfusion en continu de ladite thymosine alpha 1.

10. Utilisation selon la revendication 9, dans laquelle ladite perfusion en continu comprend une perfusion intraveineuse en continu.

11. Utilisation selon la revendication 9, dans laquelle ladite perfusion est réalisée via une minipompe.

12. Utilisation selon la revendication 10, dans laquelle ladite perfusion intraveineuse est réalisée via une minipompe.

13. Utilisation selon la revendication 11, dans laquelle ladite minipompe est une minipompe osmotique.

14. Utilisation selon la revendication 11, dans laquelle ladite minipompe est implantée dans ledit patient.

15. Utilisation selon la revendication 1, dans laquelle ledit patient est un patient cancéreux.

16. Utilisation selon la revendication 1, pour le traitement du cancer dans ledit patient.

17. Utilisation selon la revendication 1, pour le traitement du VIH dans ledit patient.

18. Utilisation selon la revendication 1, pour le traitement de l'hépatite B dans ledit patient.

19. Utilisation selon la revendication 1, pour le traitement de l'hépatite C dans ledit patient.
